# EUROPEAN PATENT SPECIFICATION

(11) **EP 3 210 648 B1**
(45) Date of publication and mention of the grant of the patent: **10.07.2019**
(21) Application number: 15853098.0
(22) Date of filing: 20.10.2015
(51) Int. Cl.: A61N 5/10, G01T 1/161

(54) **ACCURACY MANAGEMENT SYSTEM AND METHOD FOR RADIOTHERAPY APPARATUS**
GENAUIGKEITSVERWALTUNGSSYSTEM UND VERFAHREN FÜR STRAHLENTHERAPIEVORRICHTUNG
SYSTÈME DE GESTION DE LA PRÉCISION ET PROCÉDÉ POUR APPAREIL DE RADIOTHÉRAPIE

(30) Priority: 22.10.2014 KR 20140143596
(43) Date of publication of application: 30.08.2017
(73) Proprietor: Samsung Life Public Welfare Foundation, Seoul 04348 (KR)
(72) Inventor: JU, Sang Gyu, Seoul 06350 (KR)
(74) Representative: Patentanwälte Olbricht Buchhold Keulertz
(86) International application number: PCT/KR2015/011044
(87) International publication number: WO 2016/064153

(56) References cited:
- JP-A- 2003 175 119
- KR-A- 20080 044 252
- KR-A- 20090 119 241
- KR-A- 20100 086 694
- US-A1- 2007 041 497
- US-A1- 2008 258 083
- US-A1- 2014 105 355
- US-A1- 2014 105 355
- US-A1- 2014 267 697

## Description

### TECHNICAL FIELD

Embodiments of the inventive concept relate to a quality management system and method for a radiation therapy apparatus, and more particularly, to a quality management system and method for a radiation therapy apparatus that may configure a user-customized automatic quality management system in a quality management method for a radiation therapy apparatus using an image acquiring unit such as an electronic portal imaging device (EPID).

### BACKGROUND ART

Radiation therapy is a method of delaying or preventing the growth of a malignant tissue or extinguishing the malignant tissue by damaging or destroying a target tissue by using high-energy waves such as X-rays or gamma-rays or high-energy particles such as electron beams or proton beams. Radiation therapy is also used to treat benign tumors, medical illnesses, and some skin diseases as well as cancers. Recently, a radiation surgery method for treating without incision surgery by irradiating a large amount of radiation at one time has also been developed to replace a neurosurgical surgery method incising a skull.

It has recently been generalized such that about 60% or more of cancer patients may receive radiation therapy. Not only being used to treat a tumor by itself, radiation therapy may be used to reduce the size of a tumor to facilitate a surgical surgery or to destroy a malignant cell left after surgery, by being used together with other surgical surgeries for treating a local portion that fails to be removed by surgery or where surgery is difficult because a tumor is large and invasive.

Extracorporeal radiation therapy apparatuses for irradiating radiations from outside may be classified into low-energy X-ray therapy apparatuses, radioisotope therapy apparatuses, linear accelerators, particle accelerators, and the like according to the methods of generating high-energy particles or radiations.

The low-energy X-ray therapy apparatuses have been used to treat skin diseases or deep portions by using X-ray generating apparatuses, but they are rarely used nowadays.

The radioisotope therapy apparatuses use gamma-rays generated from radioisotopes such as cobalt-60 (Co-60). The radioisotope therapy apparatuses use gamma-rays having somewhat higher energy than the X-rays of the low-energy X-ray therapy apparatuses, but their use is gradually decreasing.

As apparatuses used as the standard of radiation therapy, the linear accelerators may output X-rays and electron beams, may transmit various energy, and may provide a high dose rate and beam-forming.

The particle accelerators have a structure for transferring neutron or proton particles accelerated by a cyclotron accelerator through a beam transfer pipe and emitting the same to a desired region through a nozzle, and may minimize a dose at a normal tissue and concentrate energy only on a deep tumor because they have a deeper Bragg's peak than the linear accelerators.

The U.S. patent application publication 2014/105355 A1 (TOIMELA LASSE HEIKKI ET AL), 17 April 2014, discloses a quality management system and method for a radiation therapy apparatus.

Recent medical radiation therapy apparatuses are developed in the form of mounting a radiation emitting head on a gantry having an arm or in the form of having a ring-type gantry, and a structure in which a radiation emitting head and a radiation detector face each other with a human body therebetween and rotate around the human body is mainly used. Information disclosed in this Background section was already known to the inventors before achieving the inventive concept or is technical information acquired in the process of achieving the inventive concept. Therefore, it may contain information that does not form the prior art that is already known to the public in this country.

### DETAILED DESCRIPTION OF THE INVENTION

### TECHNICAL PROBLEM

Embodiments of the inventive concept are to provide a quality management system and method for a radiation therapy apparatus that may configure a user-customized automatic quality management system in a quality management system and method for a radiation therapy apparatus using a dose measuring unit or an image acquiring unit such as an electronic portal imaging device (EPID).

### TECHNICAL SOLUTION

The invention is as defined by the appended claims.

According to an embodiment of the inventive concept, a quality management system for a radiation therapy apparatus includes: an input/output unit displaying selectable quality management items; a quality management order error determining unit determining whether there is an error in the order of the quality management items that are selected and arranged by the input/output unit; and a quality management execution control unit performing control such that quality management is executed in the order of the quality management items that are determined by the quality management order error determining unit to have no error in the arrangement order thereof.

Herein, the quality management system may further include a control unit generating a predetermined quality management module by storing the quality management items in the arrangement order of the quality management items that are determined by the quality management order error determining unit to have no error in the arrangement order thereof.

Herein, a notification may be output to notify an error in the order when there is an error in the order of the arranged quality management items as a result of the determination of the quality management order error determining unit.

Herein, the quality management system may further include a report generating unit generating and providing a result report of the quality management executed by the quality management execution control unit.

Herein, the quality management system may further include: a body unit; a gantry coupled to one side of the body unit and formed to be rotatable in at least one direction with respect to the body unit or to be independently installed; a radiation irradiating head formed at one side of the gantry to irradiate a radiation; and an image acquiring unit formed to face the radiation irradiating head to detect the radiation irradiated by the radiation irradiating and convert the detected radiation into an electrical signal to acquire image, wherein the quality management execution control unit may control the movement of at least one of the gantry, the radiation irradiating head, and the image acquiring unit to irradiate the radiation for each of the quality management items such that the quality management corresponding to each item is performed.

Herein, the image acquiring unit may include an electronic portal imaging device (EPID).

According to another embodiment of the inventive concept, a quality management method for a radiation therapy apparatus includes: displaying one or more quality management items on an input/output unit; arranging the quality management items according to a user input; determining whether there is an error in the order of the arranged quality management items; and performing control such that quality management is executed in the order of the quality management items that are determined to have no error in the arrangement order thereof.

Herein, the quality management method may further include generating a predetermined quality management module by storing the quality management items in the arrangement order of the quality management items that are determined to have no error in the arrangement order thereof.

Herein, the quality management method may further include outputting a notification to notify an error in the order when there is an error in the order of the arranged quality management items as a result of the determination.

Herein, the quality management method may further include generating and providing a result report of the executed quality management after the performing of the control such that the quality management is executed.

Herein, the performing of the control such that the quality management is executed may include controlling the movement of at least one of a gantry, a radiation irradiating head, and an image acquiring unit of the radiation therapy apparatus to irradiate a radiation for each of the quality management items such that the quality management corresponding to each item is performed.

Herein, the image acquiring unit may include an electronic portal imaging device (EPID).

These and/or other aspects will become apparent and more readily appreciated from the following description of the invention, taken in conjunction with the accompanying drawings.

### ADVANTAGEOUS EFFECTS OF THE INVENTION

By the quality management system and method for a radiation therapy apparatus according to the embodiments of the inventive concept, it is possible to construct a customized quality management automation system suitable for the real state of each hospital. Also, since customized quality management automation may be implemented in this manner, the time required for quality management may be considerably reduced from 2 days per month to 3 hours per month. Also, since the quality management may be automatically performed at night without a user, the workload of a radiation therapy apparatus or a quality manager may be reduced. Also, since the quality management results of respective quality management items performed may be analyzed and the result report thereof may be automatically generated and stored, the quality management history thereof may be systematically stored and analyzed.

### DESCRIPTION OF THE DRAWINGS

FIG. 1 is a diagram illustrating a radiation therapy apparatus according to an embodiment of the inventive concept.
FIG. 2 is a block diagram illustrating an internal configuration of the radiation therapy apparatus of FIG. 1.
FIGS. 3A and 3B are diagrams illustrating an arrangement order of quality management items.
FIG. 4 is a diagram illustrating a form in which performance result screens of respective quality management items are displayed in order.
FIG. 5 is a flow diagram illustrating a quality management method for a radiation therapy apparatus according to an embodiment of the inventive concept.

### BEST MODE

The inventive concept may include various embodiments and modifications, and certain embodiments thereof are illustrated in the drawings and will be described herein in detail. The effects and features of the inventive concept and the accomplishing methods thereof will become apparent from the following description of the embodiments, taken in conjunction with the accompanying drawings. However, the inventive concept is not limited to the embodiments described below, and may be embodied in various modes. It will be understood that although the terms "first", "second", etc. may be used herein to describe various components, these components should not be limited by these terms. These terms are only used to distinguish one component from another. Also, as used herein, the singular forms "a", "an", and "the" are intended to include the plural forms as well, unless the context clearly indicates otherwise. Also, it will be understood that the terms "comprise", "include" and "have" used herein specify the presence of stated features or components, but do not preclude the presence or addition of one or more other features or components. Also, the sizes of components in the drawings may be exaggerated for convenience of description. In other words, since the sizes and thicknesses of components in the drawings are arbitrarily illustrated for convenience of description, the following embodiments are not limited thereto.

Hereinafter, embodiments of the inventive concept will be described in detail with reference to the accompanying drawings. In the following description, like reference numerals will denote like elements, and redundant descriptions thereof will be omitted.

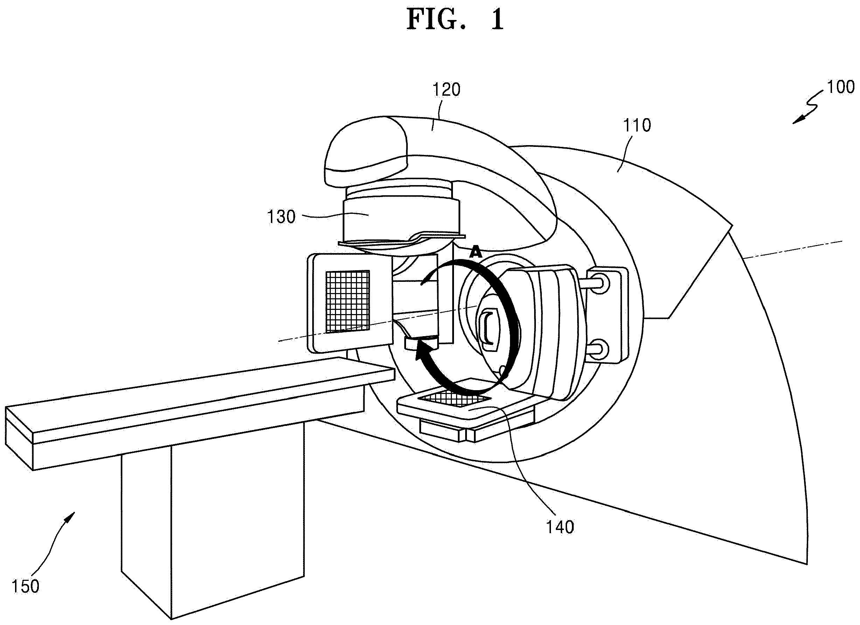

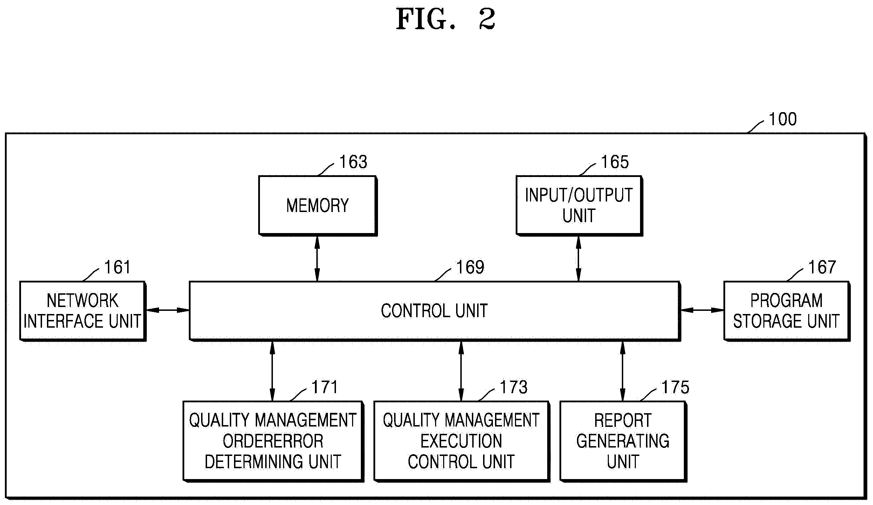

FIG. 1 is a diagram illustrating a quality management system 100 for a radiation therapy apparatus according to an embodiment of the inventive concept, and FIG. 2 is a block diagram illustrating an internal configuration of the quality management system 100 of FIG. 1.

Referring to FIGS. 1 and 2, the quality management system 100 for a radiation therapy apparatus according to an embodiment of the inventive concept may include a body unit 110, a gantry 120, a radiation irradiating head 130, an image acquiring unit 140, and a bed unit 150. Also, the quality management system 100 for a radiation therapy apparatus according to an embodiment of the inventive concept may include a network interface unit 161, a memory 163, an input/output unit 165, a program storage unit 167, a control unit 169, a quality management order error determining unit 171, a quality management execution control unit 173, and a report generation unit 175. This will be described below in more detail.

Radiation therapy is a therapy method for treating a cancer by irradiating a high-dose radiation intensively to a tumor. Successful radiation therapy requires a therapy technology for concentrating a radiation on a tumor while minimizing the damage to surrounding normal organs, an accurate radiation therapy apparatus, and various image or dose detecting apparatuses.

Recently, with the widespread use of high-accuracy radiation therapy apparatuses, high-dose irradiation using high-level therapy technology has been generalized. The high-dose irradiation has improved the tumor removal efficiency, but the potential risk of radiation accidents due to erroneous irradiation has also increased. Thus, recently, in order to prevent these radiation accidents, the strict quality management (quality assurance) of therapy apparatuses has been prescribed by law. Meanwhile, an electronic portal imaging device (EPID) has been mainly used to detect the therapy posture of a patient for the purpose of accurate therapy, but the attempt to use the EPID as a means for quality management (quality assurance) of a radiation therapy apparatus has recently increased. In quality management, the use of an EPID is convenient and highly efficient in comparison with the conventional method and requires no film because digital image acquisition is possible.

Referring to FIG. 1, the body unit 110 forms a base unit of the radiation therapy apparatus and becomes a standard for rotation of the gantry 120, the radiation irradiating head 130, and the image acquiring unit 140.

The gantry 120 may be coupled to one side of the body unit 110 and formed to be rotatable in at least one direction with respect to the body unit 110. In this case, the image acquiring unit 140 formed to face the radiation irradiating head 130 of the gantry 120 may rotate together with the gantry 120. That is, the gantry 120, the radiation irradiating head 130, and the image acquiring unit 140 may be formed to be rotatable in the direction of an arrow A in FIG. 1 (or in the opposite direction thereof).

The radiation irradiating head 130 may be formed at one side of the gantry 120 to irradiate a radiation. Herein, the radiation irradiating head 130 may emit X-rays, gamma-rays, high-energy electron beams, high-energy proton beams, or other high-energy particle beams.

Also, the radiation irradiating head 130 may include any one of an X-ray generating apparatus, a radioisotope source, and a linear accelerator. Alternatively, the radiation irradiating head 130 may receive and emit high-energy particle beams accelerated and generated by a particle accelerator installed outside the radiation therapy apparatus. Alternatively, the radiation irradiating head 130 may include a multi-leaf collimator (MLC). By using the multi-leaf collimator, the radiation irradiating head 130 may provide internal beamforming, thus enabling more efficient radiation energy transmission.

The image acquiring unit 140 may include an image sensor or a detector sensor and may detect a radiation and convert the same into an electrical signal to acquire an image. An electronic portal imaging device (EPID) may be used as an embodiment of the image acquiring unit 140. In detail, in the radiation therapy using high-energy radiation, in order to detect the position of a diseased part of a patient, the EPID technology may detect the radiation transmitted through the patient and convert the same into an electrical signal to acquire an image or dose distribution. That is, the image acquiring unit 140 may acquire a radiation image or may acquire a dose distribution.

The image acquiring unit 140 may be mounted on any one of the body unit 110, the gantry 120, and the radiation irradiating head 130. Alternatively, the image acquiring unit 140 may be independently mounted on the bed unit 150 without being mounted on the radiation therapy apparatus, or may be installed by using a separate support.

The bed unit 150 may be formed such that the patient may lie thereon, and may be configured to move in an X-axis direction, a Y-axis direction, or a Z-axis direction with respect to the radiation irradiated from the radiation irradiating head 130.

Referring to FIG. 2, the quality management system 100 for a radiation therapy apparatus according to an embodiment of the inventive concept may further include a network interface unit 161, a memory 163, an input/output unit 165, a program storage unit 167, a control unit 169, a quality management order error determining unit 171, a quality management execution control unit 173, and a report generation unit 175.

In detail, a quality management procedure for a radiation therapy apparatus is performed selectively according to the user's environments among up to about 40 types of quality management items made based on the recommendations of the American Medical Physics Society and the like. Each hospital receives preapproval for the quality management items and procedures from the national institutions and then performs quality management on a daily, weekly, monthly, or yearly basis and takes periodical tests thereon. The quality management may be generally divided into the radiation output accuracy of the therapy apparatus and the geometrical accuracy of the radiation therapy apparatus. The geometrical tests may include about 20 types of tests including the accuracy of the rotation axis (isocenter) of the radiation therapy apparatus, the accuracy of the size of an irradiation surface, the accuracy of the position of an MLC leaf, and the like.

In the case of the conventional quality management method, the manpower performs the respective quality management items in order; however, since the quality management may require high precision and high accuracy, its procedure may be difficult, it may be time-consuming, and costs may occur due to the use of consumables. Various quality management systems have recently been developed to solve these problems; however, the automation thereof has been difficult due to the quality management items varying according to apparatus models and various hospital environments.

In order to solve these problems, in the quality management system 100 for a radiation therapy apparatus according to an embodiment of the inventive concept, a program may be developed such that each of the quality management items of the radiation therapy apparatus may be performed by the EPID, and it may be modularized such that each user may easily add or rearrange modules. Accordingly, the user may construct a customized automatic quality management system by selecting necessary items among the quality management items in accordance with the user apparatus and the hospital status.

Hereinafter, the respective components of the quality management system 100 for a radiation therapy apparatus according to the inventive concept will be described in more detail.

The network interface unit 161 may provide a communication interface necessary to communicate the quality management results of the quality management system 100 with an external server in conjunction with a communication network (not illustrated). The memory 163 may temporarily store the data processed by the control unit 169.

The input/output unit 165 may include a touch-recognition display controller or various other input/output controllers. As an example, the touch-recognition display controller may provide an output interface and an input interface between the apparatus and the user. The touch-recognition display controller may communicate electrical signals with the control unit. Also, the touch-recognition display controller may display a visual output to the user, and the visual output may include texts, graphics, images, videos, or any combination thereof. The input/output unit 165 may include, for example, a display member such as a liquid crystal display (LCD) or an organic light emitting display (OLED) capable of touch recognition.

The program storage unit 167 may be mounted with control software that allows the quality management system 100 to perform an operation of receiving a quality management order and detecting an error in the order, an operation of executing quality management according to the order, and an operation of generating a result report of the executed quality management.

As a kind of central processing unit, the control unit 169 may control an overall process for performing quality management in the quality management system 100. For example, as various services, the control unit 169 may drive the control software mounted on the program storage unit 167, may control the input/output unit 165 to display the quality management items, may control the quality management order error determining unit 171 to determine whether there is an error in the order of the quality management items arranged by the user, may control the quality management execution control unit 173 to execute quality management according to the set quality management order, and may control the report generating unit 175 to generate a result report of the executed quality management.

The quality management order error determining unit 171 may determine whether there is an error in the order of the quality management items arranged by the user. In detail, under the control of the control unit 169, selectable quality management items may be displayed on the input/output unit 165 such as a touch panel. Then, the user may select desired quality management items among the displayed quality management items and arrange the selected quality management items in a desired order. However, the quality management items may be associated with each other to be performed according to a predetermined order, and for example, the quality management may be performed according to the order illustrated in Table 1 below. That is, the Jaw Field Size of Item 2 may be checked after the correspondence between Light Field and Radiation Field of Item 1 is detected.

**Table 1**

| | **Procedure** | **Tolerance** | **Note** |
|---|---|---|---|
| 1 | Coincidence of LF vs XF | ±1mm | LF : Light FieldXF : Radiation FieldG0 : Gantry 0° |
| 2 | Jaw FS | ±1mm | FS : Field SizeGO : Gantry 0° |
| 3 | MLC FS | ±1mm | FS : Field SizeGO : Gantry 0° |
| 4 | Collimator Star-shot | ϕ = 1.5mm | |
| 5 | MLC Collimator Star-shot | r < ±1mm | |
| 6 | MLC Picket Fence | ±1mm | |
| 7 | Half Beam | < 3mm | G0 : Gantry 0°G180 : Gantry 180° |
| 8 | Jaw Orthogonality &Jaw Symmetry | < 1°< 3mm | |
| 9 | Collimator Wintson-Lutz | ϕ = 1.5mm | |
| 10 | Gantry Winston-Lutz | ϕ = 1.5mm | |
| 11 | Couch Winston-Lutz | ϕ : ±1mm | |
| 12 | Gantry MLC Winston-Lutz | r < ±1mm | |

Thus, the quality management order error determining unit 171 may determine whether there is an error in the order of the quality management items arranged by the user and may notify an error in the order to the user through a warning message thereof when there is an error in the order of the quality management items arranged by the user.

Herein, the respective quality management items arranged on the input/output unit 165 may be provided to be freely added, deleted, or moved by various methods such as touch & drag or click & drag. Then, the quality management order error determining unit 171 may determine and notify an error in the order to the user whenever the quality management items are added, deleted, or moved in this manner.

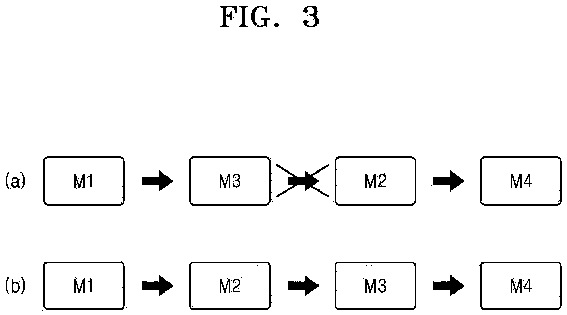

For example, when the quality management should be performed in the order of M1, M2, M3, and M4, if the user arranges the quality management items in the order of M1, M3, M2, and M4 as illustrated in FIG. 3A, the quality management order error determining unit 171 may determine whether there is an error in the order of the quality management items arranged by the user and may indicate the occurrence of an error in M3 -> M2 items that are reversely arranged. In this state, when the user arranges the quality management items in the order of M1, M2, M3, and M4 as illustrated in FIG. 3B, the quality management order error determining unit 171 may determine whether there is an error in the order of the quality management items arranged by the user and may indicate no error therein.

Meanwhile, the control unit 169 may generate a predetermined quality management module by storing the quality management items in the arrangement order of the quality management items that are determined by the quality management order error determining unit 171 to have no error in the arrangement order thereof. That is, a plurality of quality management items selected by the user and arranged in a predetermined order may be generated as one module, thus facilitating the management thereof.

The quality management execution control unit 173 may perform control such that the quality management may be executed in the order of the quality management items that are modularized after being determined by the quality management order error determining unit 171 to have no error in the arrangement order thereof. That is, the quality management execution control unit 173 may control the movement of the gantry 120, the radiation irradiating head 130, the image acquiring unit 140, and/or the bed unit 150 to irradiate the radiation for each of the quality management items such that the quality management corresponding to each item may be performed.

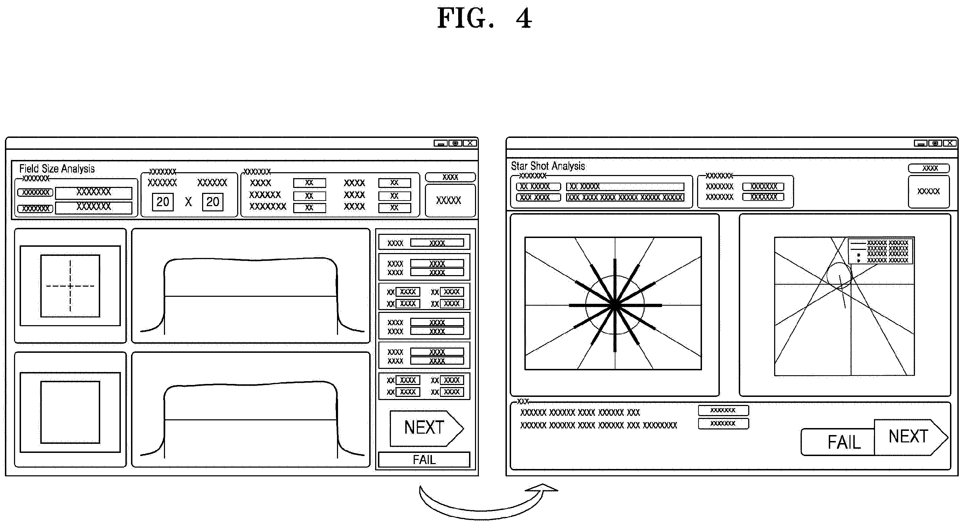

That is, as illustrated in FIG. 4 illustrating a process of executing each item of the quality management, the modularized quality management items may be performed in order, the results thereof may be displayed on the input/output unit 165, and the quality management of the next item may be automatically performed upon completion of the quality management of one item.

The report generating unit 175 may analyze the quality management results of the respective quality management items executed by the quality management execution control unit 173 and may generate a result report thereof. That is, the report generating unit 175 may automatically analyze the performance results of the respective quality management items and may provide the same to the user.

By the inventive concept described above, it is possible to construct a customized quality management automation system suitable for the real state of each hospital. Also, since customized quality management automation may be implemented in this manner, the time required for quality management may be considerably reduced from 2 days per month to 3 hours per month. Also, since the quality management may be automatically performed at night without the user, the workload of the radiation therapy apparatus or the quality manager may be reduced. Also, since the quality management results of the respective quality management items performed may be analyzed and the result report thereof may be automatically generated and stored, the quality management history thereof may be systematically stored and analyzed.

### MODE OF THE INVENTION

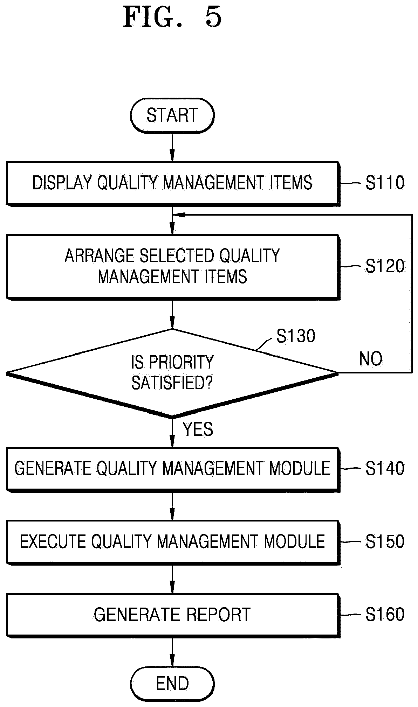

Hereinafter, a quality management method for a radiation therapy apparatus according to an embodiment of the inventive concept will be described. FIG. 5 is a flow diagram illustrating a quality management method for a radiation therapy apparatus according to an embodiment of the inventive concept.

Referring to FIG. 5, the quality management method for a radiation therapy apparatus according to an embodiment of the inventive concept may include: displaying quality management items (operation S110); arranging the quality management items selected by a user input in order (operation S120); determining whether there is no error in the order of the arranged quality management items because they satisfy the priority (operation S130); generating a quality management module (operation S140); executing the quality management module to execute quality management in the order of the quality management items (operation S150); and analyzing the quality management results of the respective quality management items performed and generating a result report thereof (operation S160). This will be described below in more detail.

First, the quality management items may be displayed (operation S110). In detail, under the control of the control unit 169, selectable quality management items may be displayed on the input/output unit 165 such as a touch panel. Then, the user may select desired quality management items among the displayed quality management items and arrange the selected quality management items in a desired order. Herein, the respective quality management items arranged on the input/output unit 165 may be freely added, deleted, or moved by various methods such as touch & drag or click & drag.

Thereafter, the quality management items selected by the user input may be arranged in order (operation S120), and it may be determined whether there is no error in the order of the arranged quality management items because they satisfy the priority (operation S130). That is, the quality management order error determining unit 171 may determine whether there is an error in the order of the quality management items arranged by the user and may notify an error in the order to the user through a warning message thereof when there is an error in the order of the quality management items arranged by the user.

Thereafter, the quality management module may be generated (operation S140). That is, the control unit 169 may generate a predetermined quality management module by storing the quality management items in the arrangement order of the quality management items that are determined by the quality management order error determining unit 171 to have no error in the arrangement order thereof. That is, a plurality of quality management items selected by the user and arranged in a predetermined order may be generated as one module, thus facilitating the management thereof.

Thereafter, the quality management module may be executed to execute the quality management in the order of the quality management items (operation S150). In detail, the quality management execution control unit 173 may perform control such that the quality management may be executed in the order of the quality management items that are modularized after being determined by the quality management order error determining unit 171 to have no error in the arrangement order thereof. That is, the quality management execution control unit 173 may control the movement of the gantry 120, the radiation irradiating head 130, the image acquiring unit 140, and/or the bed unit 150 to irradiate the radiation for each of the quality management items such that the quality management corresponding to each item may be performed.

Thereafter, the quality management results of the respective quality management items performed may be analyzed and the result report thereof may be generated (operation S160). In detail, the report generating unit 175 may analyze the quality management results of the respective quality management items executed by the quality management execution control unit 173 and may generate the result report thereof. That is, the report generating unit 175 may automatically analyze the performance results of the respective quality management items and may provide the same to the user.

By the inventive concept described above, it is possible to construct a customized quality management automation system suitable for the real state of each hospital. Also, since customized quality management automation may be implemented in this manner, the time required for quality management may be considerably reduced from 2 days per month to 3 hours per month. Also, since the quality management may be automatically performed at night without the user, the workload of the radiation therapy apparatus or the quality manager may be reduced. Also, since the quality management results of the respective quality management items performed may be analyzed and the result report thereof may be automatically generated and stored, the quality management history thereof may be systematically stored and analyzed.

The embodiments of the inventive concept described above may be implemented in the form of computer programs that may be executed through various components on a computer, and the computer programs may be recorded in computer-readable recording mediums. Examples of the computer-readable recording mediums may include magnetic recording mediums such as hard disks, floppy disks, and magnetic tapes, optical recording mediums such as CD-ROMs and DVDs, magneto-optical recording mediums such as floptical disks, and hardware devices such as ROMs, RAMs and flash memories that are especially configured to store and execute program commands. Also, the computer-readable recording mediums may include intangible mediums that are implemented in an intangible form transmittable on a network, and may be, for example, mediums that are implemented in the form of software or application and then may be transmitted and distributed over a network.

The computer programs may be those that are especially designed and configured for the inventive concept, or may be those that are known and available to computer programmers skilled in the art. Examples of the computer programs may include machine language codes that may be generated by a compiler, and high-level language codes that may be executed by a computer by using an interpreter.

Particular implementations described herein are merely embodiments, and do not limit the scope of the inventive concept in any way. For the sake of conciseness, descriptions of related art electronic configurations, control systems, software, and other functional aspects of the systems may be omitted. Also, the connection lines or connection members between various elements illustrated in the drawings represent examples of functional connections and/or physical or logical connections between the various elements, and various alternative or additional functional connections, physical connections, or logical connections may be present in practical apparatuses. Also, no element may be essential to the practice of the inventive concept unless the element is specifically described as "essential" or "critical".

Thus, the scope of the inventive concept is not limited to the above embodiments, and the scope of the inventive concept may include both the following claims and the equivalents thereof.

### INDUSTRIAL APPLICABILITY

The embodiments of the inventive concept may be used in the quality management system and method for the radiation therapy apparatus.

## Claims

1. A quality management system (100) for a radiation therapy apparatus, the quality management system comprising:
an input/output unit (165) configured to display selectable quality management items and configured to allow a user to select desired quality management items among the displayed quality management items and arrange the selected quality management items in a desired order; f
a quality management order error determining unit (171) configured to determine whether there is an error in the order of the quality management items that are selected and arranged in a desired order by the user; and
a quality management execution control unit (173) configured to perform control such that quality management is executed in the order of the quality management items that are determined by the quality management order error determining unit to have no error in the arrangement order thereof.

2. The quality management system of claim 1, further comprising a control unit generating a predetermined quality management module by storing the quality management items in the arrangement order of the quality management items that are determined by the quality management order error determining unit to have no error in the arrangement order thereof.

3. The quality management system of claim 1, wherein a notification is output to notify an error in the order when there is an error in the order of the arranged quality management items as a result of the determination of the quality management order error determining unit.

4. The quality management system of claim 1, further comprising a report generating unit generating and providing a result report of the quality management executed by the quality management execution control unit.

5. The quality management system of claim 1, further comprising:
a body unit;
a gantry coupled to one side of the body unit and formed to be rotatable in at least one direction with respect to the body unit or to be independently installed;
a radiation irradiating head formed at one side of the gantry to irradiate a radiation; and
an image acquiring unit formed to face the radiation irradiating head to detect the radiation irradiated by the radiation irradiating and convert the detected radiation into an electrical signal to acquire image, wherein the quality management execution control unit controls the movement of at least one of the gantry, the radiation irradiating head, and the image acquiring unit to irradiate the radiation for each of the quality management items such that the quality management corresponding to each item is performed.

6. The quality management system of claim 5, wherein the image acquiring unit comprises an electronic portal imaging device (EPID).

7. A quality management method for a radiation therapy apparatus, the quality management method being carried out by a computer and comprising the steps of:
displaying one or more quality management items on an input/output unit;
arranging the quality management items according to a user input;
determining whether there is an error in the order of the arranged quality management items; and
performing control such that quality management is executed in the order of the quality management items that are determined to have no error in the arrangement order thereof.

8. The quality management method of claim 7, further comprising generating a predetermined quality management module by storing the quality management items in the arrangement order of the quality management items that are determined to have no error in the arrangement order thereof.

9. The quality management method of claim 7, further comprising outputting a notification to notify an error in the order when there is an error in the order of the arranged quality management items as a result of the determination.

10. The quality management method of claim 7, further comprising generating and providing a result report of the executed quality management after the performing of the control such that the quality management is executed.

11. The quality management method of claim 7, wherein the performing of the control such that the quality management is executed comprises controlling the movement of at least one of a gantry, a radiation irradiating head, and an image acquiring unit of the radiation therapy apparatus to irradiate a radiation for each of the quality management items such that the quality management corresponding to each item is performed.

12. The quality management method of claim 11, wherein the image acquiring unit comprises an electronic portal imaging device (EPID).

13. A computer program stored in a medium and comprising instructions which, when the program is executed by a computer, cause the computer to execute the quality management method of claim 7.

## Patentansprüche

1. Qualitätsverwaltungssystem (100) für eine Strahlentherapievorrichtung, wobei das Qualitätsverwaltungssystem umfasst:
eine Eingabe-/Ausgabeeinheit (165), die konfiguriert ist, um auswählbare Qualitätsverwaltungspunkte anzuzeigen und die konfiguriert ist, um es einem Benutzer zu ermöglichen, gewünschte Qualitätsverwaltungspunkte aus den angezeigten Qualitätsverwaltungspunkten auszuwählen und die ausgewählten Qualitätsverwaltungspunkte in einer gewünschten Reihenfolge anzuordnen;
eine Reihenfolgenfehlerbestimmungseinheit der Qualitätsverwaltung (171), die konfiguriert ist, um zu bestimmen, ob in der Reihenfolge der Qualitätsverwaltungspunkte, die vom Benutzer ausgewählt und in einer gewünschten Reihenfolge angeordnet werden, ein Fehler vorliegt; und
eine Ausführungssteuerungseinheit der Qualitätsverwaltung (173), die konfiguriert ist, um eine Steuerung durchzuführen, sodass Qualitätsverwaltung in der Reihenfolge der Qualitätsverwaltungspunkte durchgeführt wird, bei denen von der Reihenfolgenfehlerbestimmungseinheit der Qualitätsverwaltung bestimmt wird, dass sie keinen Fehler in der Anordnungsreihenfolge davon aufweisen.

2. Qualitätsverwaltungssystem nach Anspruch 1, weiter umfassend eine Steuerungseinheit, die ein vorbestimmtes Qualitätsverwaltungsmodul durch Speichern der Qualitätsverwaltungspunkte in der Anordnungsreihenfolge der Qualitätsverwaltungspunkte erzeugt, bei denen von der Reihenfolgenfehlerbestimmungseinheit der Qualitätsverwaltung bestimmt wird, dass sie keinen Fehler in der Anordnungsreihenfolge davon aufweisen.

3. Qualitätsverwaltungssystem nach Anspruch 1, wobei eine Mitteilung ausgegeben wird, um einen Fehler in der Reihenfolge auszugeben, wenn als ein Ergebnis der Bestimmung der Reihenfolgenfehlerbestimmungseinheit der Qualitätsverwaltung ein Fehler in der Reihenfolge der angeordneten Qualitätsverwaltungspunkte vorliegt.

4. Qualitätsverwaltungssystem nach Anspruch 1, weiter umfassend eine Berichterzeugungseinheit, die einen Ergebnisbericht der Qualitätsverwaltung, die von der Ausführungssteuerungseinheit der Qualitätsverwaltung ausgeführt wird, erzeugt und bereitstellt.

5. Qualitätsverwaltungssystem nach Anspruch 1, weiter umfassend:
eine Körpereinheit;
ein Gestell, das an einer Seite der Körpereinheit gekoppelt ist und gebildet ist, um in Bezug auf die Körpereinheit in mindestens eine Richtung drehbar zu sein oder um unabhängig installiert zu werden;
einen strahlenausstrahlenden Kopf, der an einer Seite des Gestells gebildet ist, um einen Strahlen auszustrahlen; und
eine Bilderfassungseinheit, die gebildet ist, um dem strahlenausstrahlenden Kopf zugewandt zu sein, um den Strahlen, der vom Strahlenausstrahlen ausgestrahlt wird, zu detektieren und den detektierten Strahlen zum Bilderfassen in ein elektrisches Signal umzuwandeln, wobei die Ausführungssteuerungseinheit der Qualitätsverwaltung die Bewegung von mindestens einem von dem Gestell, dem strahlenausstrahlenden Kopf und der Bilderfassungseinheit steuert, um den Strahlen für jeden der Qualitätsverwaltungspunkte auszustrahlen, sodass die Qualitätsverwaltung entsprechend jedem Punkt durchgeführt wird.

6. Qualitätsverwaltungssystem nach Anspruch 5, wobei die Bilderfassungseinheit eine elektronische Portalbildgebungsvorrichtung (EPID) umfasst.

7. Qualitätsverwaltungsverfahren für eine Strahlentherapievorrichtung, wobei das Qualitätsverwaltungsverfahren von einem Computer vorgenommen wird und die folgenden Schritte umfasst:
Anzeigen einer oder mehrerer Qualitätsverwaltungspunkte auf einer Eingabe-/Ausgabeeinheit;
Anordnen der Qualitätsverwaltungspunkte gemäß einer Benutzereingabe;
Bestimmen, ob ein Fehler in der Reihenfolge der angeordneten Qualitätsverwaltungspunkte vorliegt; und
Durchführen einer Steuerung, sodass Qualitätsverwaltung in der Reihenfolge der Qualitätsverwaltungspunkte durchgeführt wird, bei denen bestimmt wird, dass sie keinen Fehler in der Anordnungsreihenfolge davon aufweisen.

8. Qualitätsverwaltungsverfahren nach Anspruch 7, weiter umfassend Erzeugen eines vorbestimmten Qualitätsverwaltungsmoduls durch Speichern der Qualitätsverwaltungspunkte in der Anordnungsreihenfolge der Qualitätsverwaltungspunkte, bei denen bestimmt wird, dass sie keinen Fehler in der Anordnungsreihenfolge davon aufweisen.

9. Qualitätsverwaltungsverfahren nach Anspruch 7, weiter umfassend Ausgeben einer Mitteilung, um einen Fehler in der Reihenfolge mitzuteilen, wenn als ein Ergebnis der Bestimmung ein Fehler in der Reihenfolge der angeordneten Qualitätsverwaltungspunkte vorliegt.

10. Qualitätsverwaltungsverfahren nach Anspruch 7, weiter umfassend Erzeugen und Bereitstellen eines Ergebnisberichts der ausgeführten Qualitätsverwaltung nach dem Durchführen der Steuerung, sodass die Qualitätsverwaltung ausgeführt wird.

11. Qualitätsverwaltungsverfahren nach Anspruch 7, wobei das Durchführen der Steuerung, sodass die Qualitätsverwaltung ausgeführt wird, ein Steuern der Bewegung von mindestens einem von einem Gestell, einem strahlenausstrahlenden Kopf und einer Bilderfassungseinheit der Strahlentherapievorrichtung umfasst, um einen Strahlen für jeden der Qualitätsverwaltungspunkte auszustrahlen, sodass die Qualitätsverwaltung entsprechend jedem Punkt durchgeführt wird.

12. Qualitätsverwaltungsverfahren nach Anspruch 11, wobei die Bilderfassungseinheit eine elektronische Portalbildgebungsvorrichtung (EPID) umfasst.

13. Computerprogramm, das in einem Medium gespeichert ist und Anweisungen umfasst, die, wenn das Programm von einem Computer ausgeführt wird, den Computer dazu veranlassen, das Qualitätsverwaltungsverfahren nach Anspruch 7 auszuführen.

## Revendications

1. Système de gestion de la qualité (100) pour un appareil de radiothérapie, le système de gestion de la qualité comprenant :
une unité d'entrée/sortie (165) configurée pour afficher des éléments de gestion de la qualité sélectionnables et configurée pour permettre à un utilisateur de sélectionner des éléments de gestion de la qualité souhaités parmi les éléments de gestion de la qualité affichés et d'agencer les éléments de gestion de la qualité sélectionnés dans un ordre souhaité ;
une unité de détermination d'erreur d'ordre de gestion de la qualité (171) configurée pour déterminer s'il existe une erreur dans l'ordre des éléments de gestion de la qualité qui sont sélectionnés et agencés dans un ordre souhaité par l'utilisateur ; et
une unité de commande d'exécution de gestion de la qualité (173) configurée pour réaliser une commande de telle manière que la gestion de la qualité est exécutée dans l'ordre des éléments de gestion de la qualité qui sont déterminés par l'unité de détermination d'erreur d'ordre de gestion de la qualité pour n'obtenir aucune erreur dans l'ordre d'agencement de ceux-ci.

2. Système de gestion de la qualité selon la revendication 1, comprenant en outre une unité de commande générant un module prédéterminé de gestion de la qualité par stockage des éléments de gestion de la qualité dans l'ordre d'agencement des éléments de gestion de la qualité qui sont déterminés par l'unité de détermination d'erreur d'ordre de gestion de la qualité pour n'obtenir aucune erreur dans l'ordre d'agencement de ceux-ci.

3. Système de gestion de la qualité selon la revendication 1, dans lequel une notification est délivrée en sortie pour notifier une erreur dans l'ordre lorsqu'il existe une erreur dans l'ordre des éléments de gestion de la qualité agencés suite à la détermination de l'unité de détermination d'erreur d'ordre de gestion de la qualité.

4. Système de gestion de la qualité selon la revendication 1, comprenant en outre une unité de génération de rapport générant et fournissant un rapport de résultat de la gestion de la qualité exécutée par l'unité de commande d'exécution de gestion de la qualité.

5. Système de gestion de la qualité selon la revendication 1, comprenant en outre :
une unité de corps ;
un portique couplé à un côté de l'unité de corps et formé de façon à pouvoir tourner dans au moins une direction par rapport à l'unité de corps ou à être installé de façon indépendante ;
une tête d'irradiation de rayonnement formée au niveau d'un côté du portique pour irradier un rayonnement ; et
une unité d'acquisition d'image formée pour être face à la tête d'irradiation de rayonnement pour détecter le rayonnement irradié par le rayonnement irradiant et convertir le rayonnement détecté en un signal électrique pour acquérir une image, dans lequel l'unité de commande d'exécution de gestion de la qualité commande le mouvement d'au moins l'un du portique, de la tête d'irradiation de rayonnement et de l'unité d'acquisition d'image pour irradier le rayonnement pour chacun des éléments de gestion de la qualité de telle manière que la gestion de la qualité correspondant à chaque élément est réalisée.

6. Système de gestion de la qualité selon la revendication 5, dans lequel l'unité d'acquisition d'image comprend un dispositif d'imagerie portale électronique (EPID).

7. Procédé de gestion de la qualité pour un appareil de radiothérapie, le procédé de gestion de la qualité étant mis en oeuvre par un ordinateur et comprenant les étapes de :
affichage d'un ou plusieurs éléments de gestion de la qualité sur une unité d'entrée/sortie ;
agencement des éléments de gestion de la qualité en fonction d'une entrée utilisateur ;
fait de déterminer s'il existe une erreur dans l'ordre des éléments de gestion de la qualité agencés ; et
réalisation d'une commande de telle manière que la gestion de la qualité est exécutée dans l'ordre des éléments de gestion de la qualité qui sont déterminés pour n'obtenir aucune erreur dans l'ordre d'agencement de ceux-ci.

8. Procédé de gestion de la qualité selon la revendication 7, comprenant en outre la génération d'un module prédéterminé de gestion de la qualité par stockage des éléments de gestion de la qualité dans l'ordre d'agencement des éléments de gestion de la qualité qui sont déterminés pour n'obtenir aucune erreur dans l'ordre d'agencement de ceux-ci.

9. Procédé de gestion de la qualité selon la revendication 7, comprenant en outre la délivrance en sortie d'une notification pour notifier une erreur dans l'ordre lorsqu'il existe une erreur dans l'ordre des éléments de gestion de la qualité agencés suite à la détermination.

10. Procédé de gestion de la qualité selon la revendication 7, comprenant en outre la génération et la fourniture d'un rapport de résultat de la gestion de la qualité exécutée après la réalisation de la commande de telle manière que la gestion de la qualité est exécutée.

11. Procédé de gestion de la qualité selon la revendication 7, dans lequel la réalisation de la commande de telle manière que la gestion de la qualité est exécutée comprend la commande du mouvement d'au moins l'un d'un portique, d'une tête d'irradiation de rayonnement et d'une unité d'acquisition d'image de l'appareil de radiothérapie pour irradier un rayonnement pour chacun des éléments de gestion de la qualité de telle manière que la gestion de la qualité correspondant à chaque élément est réalisée.

12. Procédé de gestion de la qualité selon la revendication 11, dans lequel l'unité d'acquisition d'image comprend un dispositif d'imagerie portale électronique (EPID).

13. Programme informatique stocké dans un support et comprenant des instructions qui, lorsque le programme est exécuté par un ordinateur, amènent l'ordinateur à exécuter le procédé de gestion de la qualité selon la revendication 7.
